# EUROPEAN PATENT APPLICATION

(11) **EP 0 761 243 A1**
(43) Date of publication of application: **12.03.1997**
(21) Application number: 96306544.6
(22) Date of filing: 09.09.1996
(51) Int. Cl.: A61L 29/00, A61L 31/00

(54) **Biostatic coatings and processes**

(30) Priority: 08.09.1995 US 3437
(71) Applicant: UNION CARBIDE CHEMICALS AND PLASTICS COMPANY, INC., Danbury Connecticut 06817-0001 (US)
(72) Inventor: Fan, You Ling, East Brunswick, New Jersey 08816 (US)
(74) Representative: Allard, Susan Joyce

(57) **Abstract**

Hydrophilic, biostatic coatings suitable for coating medical devices are disclosed. The hydrophilic coatings comprise an antimicrobial agent selected from halogenated hydroxy or acyloxy diphenyl ethers. Surprisingly, the coatings exhibit excellent biostatic activities against many common infectious microorganisms even after prolonged durations.

## Description

### Field of the Invention

This invention relates to coatings which are hydrophilic and biostatic and to processes for applying such coatings to substrates, e.g., medical devices.

### Background of the Invention

A significant portion of hospital-acquired infections results from catheterization procedures. The most common infectious bacteria include Staphylococcus epidermis, Staphylococcus aureus, Escherichia coli, Proteus mirabilis, among others. Many of these infections are difficult to treat with antibiotics and often necessitate the removal of the medical device which is traumatic to the patient and increases the medical cost. Furthermore, encrustation of medical devices is a serious problem associated with catherization procedures performed in the urinary and G. I. tracks. The mineralization process is often accompanied with bacteria colonization which necessitates the removal of the implanted device. The presence of encrust on the surfaces of a device also makes its removal from a patient more difficult and painful. Consequently, biostatic coatings, suitable for application to such medical devices which are effective in preventing or reducing the colonization of any of these infectious bacteria on the surfaces of medical devices would be highly desirable.

A number of antimicrobial agents have been investigated for their ability to control infection by incorporation into medical devices. For instance, Trooskin et. al. (1985) found a lower rate of catheter contamination in laboratory animals with noncovalent bonding of antibiotics to catheters. Reduced microbial adherence on medical devices was reported by incorporating antimicrobial agents such as chlorhexidine (Brook, Douglas, and Van Noort, 1986), silver oxide (Schaeffer, Story, and Johnson, 1988), silver sulfadiazine and chlorhexadine (Arrow International), Iodine-Povidone® complex (C. R. Bard), benzalkonium chloride (Tebbs and Elliott, 1993) onto medical devices. In general, these antimicrobial agents suffer from either or both of two shortcomings: (1) they exhibit relatively high MIC (minimum inhibitory concentration) for controlling these infectious bacteria; consequently, their biocidal activities don't last, and (2) many of these antimicrobial agents show unacceptable cell toxicity at the MIC levels needed to control the infectious bacteria.

Another antimicrobial agent, 2,4,4,'-trichloro-2'-hydroxydiphenyl ether (a commercially available antimicrobial agent produced by Ciba-Geigy having the trade name of either Irgasan DP 300 or Triclosan®), is reported to have been molded into plastics to make them self-disinfecting (Kingston, Seal, and Hill, 1986). This approach is limited by the high molding temperatures required for many high performance plastics which exceed the thermal tolerance of the agent. Additionally, the effectiveness of such a composition is further limited by the rate of diffusion of the agent through the plastic matrices. A further concern is the potential adverse effect of incorporating an antimicrobial agent to the physical and mechanic properties of the plastic devices. Consequently, there is a need for an antimicrobial coating for substrates such as medical devices which would retain its bioefficacy for a long duration, be lubricious and applicable to a broad scope of materials.

### Summary of the Invention

By the present invention it has been found, quite unexpectedly, that a when halogenated hydroxy or acyloxy diphenyl ether ("HDPE") is incorporated into a hydrophilic coating, the resultant coating can exhibit a combination of desirable properties for the control of the above-mentioned infectious microorganisms. The more important attributes of the HDPE-containing hydrophilic coatings are: (1) the coating often exhibits a lasting bioefficacy against many of the common infectious microorganisms; (2) the coating often exhibits a very low degree of cell toxicity in comparison to the more conventional antimicrobial agents mentioned above, (3) the coating composition can be used on a variety of substrates including plastics, elastomers, metals, and ceramics, (4) the hydrophilic surfaces often make bacteria attachment more difficult; and (5) the coating is preferably lubricious which makes the insertion and removal of the medical device much easier and minimizes potential injury to the body.

### Detailed Description of the Invention

As used herein, the term "biostatic" means a compound which kills bacteria in contact with the hydrophilic coating or the immediate vicinity of the coating but which does not have a systemic effect, i.e., affecting the body generally.

The antimicrobial agent of the present invention is derived from a halogenated 2-hydroxy diphenyl ether or a halogenated 2-acyloxy-diphenyl ether. It is incorporated into the hydrophilic coating where the antimicrobial agent is released at a preferably safe but effective rate to control the growth of many common infectious microorganisms on the surfaces of medical devices. The antimicrobial agent is incorporated into a suitable hydrophilic coating composition such that the medical devices preferably provide hydrophilic, lubricious, and biostatic surfaces which are effective in reducing bacteria colonization.

The antimicrobial agent is preferably either a halogenated 2-hydroxy diphenyl ether or a halogenated 2-acyloxy-diphenyl ether as described by Formula 1 in U.S. Patent 3,629,477 which is set forth below. 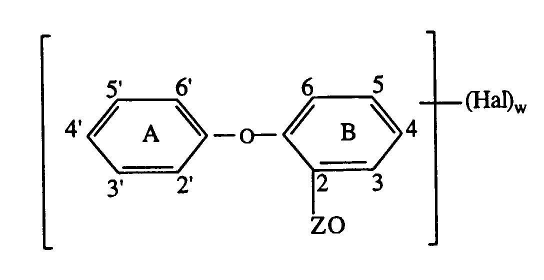 as the active ingredient against microorganisms. In this Formula I, each Hal represents identical or different halogen atoms, Z represents hydrogen or an acyl group, and
w represents a positive whole number ranging from 1 to 5, and each of the benzene rings, but preferably ring A can also contain one or several lower alkyl groups which may be halogenated, a lower alkoxy group, the allyl group, the cyano group, the amino group or lower alkanoyl group.

Preferably the methyl or methoxy group are meant by lower alkyl and lower alkoxy groups, respectively, as substituents in the benzene rings; as lower halogenated alkyl group the trifluoromethyl group is preferred.

A biocidal action similar to that of the halogen-o-hydroxy-diphenyl ethers of Formula I is also attained using the O-acyl derivatives thereof which partially or completely hydrolize under the conditions for use in practice. The esters of acetic acid, chloroacetic acid, methyl or dimethyl carbamic acid, benzoic acid, chlorobenzoic acid, methylsulfonic acid and chloromethylsulfonic acid are particularly suitable.

The polymers which comprise the coating of this invention include any water-soluble or water-swellable synthetic or naturally-occurring polymer which can be applied by any conventional coating process to provide an adherent, hydrophilic coating. As used herein, the term "water-swellable" means a substantially hydrophilic polymer which, even though is not soluble in water, would absorb sufficient water to render it lubricious in the hydrated state. In addition, the term "hydrophilic" as used herein means that water droplets do not readily form beads on the surface of such hydrophilic material, but instead, the water droplets tend to assume a contact angle of less than 45° and readily spread on its surface.

Exemplary polymers include:
I. Synthetic Water-Soluble or Water-Swellable Polymers
   1. Polyacrylates such as poly(acrylic acid), poly(hydroxyethyl acrylate), poly(dimethylamino-ethyl acrylate), and their copolymers.
   2. Poly(vinyl alcohol) and their copolymers with vinyl acetate.
   3. Poly(ethylene oxide), poly(ethylene glycol), and their copolymers with poly(propylene oxide) and poly(propylene glycol), respectively.
   4. Maleic anhydride polymers such as (maleic anhydride-methyl vinyl ether)copolymer.
   5. Polyacrylamides and their copolymers.
   6. Poly(vinyl lactam) such as poly(vinyl pyrrolidone) and its copolymers.
   7. Poly(ethylene imine).
   8. Poly(styrene sulfonate) and its copolymers.
   9. Water-soluble nylon.
   10. Poly(methacrylamidopropyltrimethylammonium chloride) and its copolymers.
   11. Poly(2-acryloamido-2-methylpropanesulfonate) and its copolymers.
   12. Polymeric complexes such as poly(acrylic acid)-poly(ethylene oxide) complex, and poly(acrylic acid)-poly(vinyl pyrrolidone) complex.
II. Nature-Occurring Polymers
   1. Cellulosic polymers such as carboxymethyl cellulose, hydroxyethyl cellulose, hydroxypropyl cellulose, Polymer JR® and LR® (trade names for quaternized cellulosic polymers produced by Union Carbide Corporation).
   2. Polysaccharides such as gum guar, alginic acid, gum arabic, chitosan, hyaluronic acid.
   3. Starches such as carboxymethyl starch, dialdehyde starch.
   4. Proteins such as gelatin, and collagen.

Those skilled in the art will recognize that water-soluble and water-swellable derivatives of the above-identified polymers can also be used in accordance with the present invention. Further details concerning the selection and application of suitable coating chemicals are known to those skilled in the art.

When the coating is applied onto medical devices made of hydrophobic materials, it is often required to impart an adhesive property to the surfaces to achieve an adherent coating. This may be accomplished by a number of methods including for example: (1) using a binder polymer, e.g., a reactive primer such as a polyisocyanate or a silane coupling agent, (2) plasma surface treatment or is-situ plasma polymerization, (3) addition of a compatible hydrophobic polymer, and (3) surface etching with chemical agents among others known to those skilled in the art. A preferred method is the use of a polyisocyanate primer as described in U.S. Patent 5,091,205.

In addition to the binder polymers and the hydrophilic polymers, the coatings of the present invention may comprise one or more additives normally used in coating formulations such as, for example, surfactants, preservatives, viscosity modifiers, pigments, dyes, antiblocking agents, and other additives known to those skilled in the art. Additionally, other functional additives which are ionically bonded to the hydrophilic polymer may also be used. These additives include ingredients such as, for example, therapeutic agents and antithrombogenic agents.

In the coating processes of the present invention, the binder polymers and the hydrophilic polymers may be delivered from liquids contained in either a solution, a dispersion or an emulsion of the polymers. In the one-step coating methods, the binder polymers and the hydrophilic polymers are contained in the same liquid medium. In the two-step methods, the binder polymers and the hydrophilic polymers are contained in separate liquid mediums. Additional coating steps may also be employed to introduce different polymers or additives. The liquid mediums used for delivering the binder polymers and hydrophilic polymers may be organic, aqueous or an organic-aqueous mixture. The liquid medium used for delivering the binder polymer can be selected so that it has some solvency for the substrate, i.e., when the substrate is polymeric. This can enhance the adhesion between the binder polymer and the substrate and aid to the film formation of the coating material. Preferred liquid mediums for delivering the binder polymers and hydrophilic polymers include, but are not limited to, esters, e.g., ethyl acetate, isopropyl acetate; ethyl acetate; alcohols, e.g., isopropyl alcohol, ethanol, butanol; ketones, e.g., acetone, methylethylketone, diacetone alcohol, methyl isobutyl ketone; amides such as dimethyl formamide; toluene; glycol ethers such as butyl glycol ether; chlorinated solvents such as dichloroethane, water, and mixtures thereof. Preferably, the liquid mediums are selected so that the binder polymers and hydrophilic polymer evenly wet the surface of the substrate to be coated.

Preferably, the concentration of the binder polymer and the hydrophilic polymers in the liquid mediums are sufficient to provide the desired amounts of the respective polymers in the coatings. Typically, the concentration of the binder polymers in the liquid medium will range from about 0.05 to 10 weight percent and, preferably, from about 0.2 to 2 weight percent based on the total weight of the liquid medium. Typically, the concentration of the hydrophilic polymers will range from about 0.1 to 20 weight percent and, preferably, from about 0.5 to 5 weight percent, based upon the total weight of the liquid medium. Further details concerning the selection of liquid mediums for delivering the binder polymers and hydrophilic polymers of the present invention are known to those skilled in the art.

Preferably, the concentration of the antimicrobial agent in the liquid medium is sufficient to provide the desired amounts of the antimicrobial acitvity in the coating. Typically, the concentration of the antimicrobial agent in the liquid medium will range from about 0.001 to 10 weight percent and, preferably, from about 0.002 to 5 weight percent based on the total weight of the liquid medium. The concentration of the antimicrobial agent in the cured coatings will typically range from about 0.2 to 80 weight percent, preferably from about 1 to 50 weight percent based on the total weight of the coating, i.e., solids content of the liquid mediums.

The antimicrobial agent may be incorporated into the coating formulation by a variety of methods to achieve comparable performance. For instance, it may be incorporated into the coating as either a solution or a dispersion or emulsion. It may be incorporated into the coating either before, or together, or after the application of the hydrophilic polymer. It is preferred that the antimicrobial agent be incorporated and applied together with the hydrophilic polymer to achieve a more uniform distribution in the coating matrix.

The coating processes of the present invention are preferably conducted in a liquid phase at atmospheric pressure and at a temperature from about 10 to 90°C. The residence times for contacting the surface of the substrate to be coated with the liquid mediums containing the binder polymer or the hydrophilic polymer, or both, range from about 1 second to 30 minutes, preferably from about 10 seconds to 10 minutes. It is generally desirable to dry the coatings after application of the coating at a temperature from about 20 to 150°C, preferably in a forced-air oven. Microwave ovens and infrared heaters may also be used if desired. Typical drying times range from about 1 minute to 24 hours and preferably range from about 10 minutes to 5 hours. When a two-step coating process is employed, it is preferred to dry the binder polymer before application of the hydrophilic polymer. When a coating composition contains one or more unsaturated monomers or prepolymers, the curing process may be effected by either ultraviolet, electron beam, gamma ray or other suitable radiation source.

The lubricious coatings which result from the coating processes of the present invention typically have a thickness of from about 0.05 to 10 microns, and preferably from about 0.1 to about 5 microns. When a two-step coating process is employed, the resulting coating preferably comprises an inner layer which is rich, i.e., greater than 50%, in the binder polymer which contacts the surface of the substrate, and an outer layer which is rich, i.e., greater than 50%, in the hydrophilic polymer which contacts the inner layer. The outer layer, which is rich in the hydrophilic polymer, has an outer surface which preferably becomes lubricious when exposed to an aqueous liquid. When a one-step coating process is employed, the resulting coating comprises a single layer which is preferably a substantially homogeneous mixture of the binder polymer and the hydrophilic polymer. However, since the binder polymer will often have more affinity for the substrate than the hydrophilic polymer, it is believed that there may be a higher concentration of the binder polymer near the surface of the substrate.

The biostatic coating of the present invention can provide a number of significant advantages over the "self-disinfecting plastic" of the prior art. First, the coating is independent of the material of construction of the device to be applied, and is therefore broadly useful on a variety of substrates. Second, the antimicrobial agent is readily available at the outer surfaces of the device where micro-organisms colonization is of the most concern. Third, the release rate of the antimicrobial agent, and hence, the bioefficacy of the device, does not vary with the material of construction of the device; it is controlled primarily by the solubility of the antimicrobial agent in the water-swollen coating matrix. Fourth, the hydrophilic coatings are preferably lubricious in the presence of aqueous fluids, e.g., body fluids, which renders microorganisms attachment to the substrate more difficult. Fifth, the presence of the biostatic coating preferably does not adversely affect the bulk properties of the substrate material. Fifth, the biostatic coating of this invention may reduce encrustation build-up on surfaces and hence prolong the useful life of a medical device. Consequently, this invention provides a predictable, sustained, and safe release of the antimicrobial agent on a variety of substrates regardless of their materials and methods of construction.

The biostatic coating of this invention may be used on any medical devices where control of microorganism colonization is desired. Exemplary medical devices suitable for this purpose include urinary and ureteral stents, Foley catheters, central venous catheters, infusion catheters, endotracheal tubes, introducers, drain tubes, instruments, wound dressings, pace-maker leads, among others. The medical devices, as well as other substrates to which the coatings of the present invention can be applied include those selected from the group consisting of polyurethane, poly(vinyl chloride), polyacrylate, polycarbonate, polystryrene, polyester resins, polybutadiene-styrene copolymers, nylon, polyethylene, polypropylene, polybutylene, silicon, poly(vinyl acetate), polymethacrylate, polysulfone, polyisoprene, copolymers and derivatives thereof, glass, metal, ceramic and mixtures thereof.

The following examples illustrate the formulation, coating process, and bioefficacy of the biostatic coating of this invention and are not intended to limit the scope of the claims which follow. Unless otherwise indicated, the chemicals used in the Examples were standard reagents and are readily commercially available.

### Example 1.

This example illustrates the preparation of a coating solution containing both a high molecular weight water-soluble polymer and 2,4,4-trichloro-2'-hydroxydiphenyl ether. Into a 2-liter stainless steel reactor, equipped with a turbine agitator, condenser, thermometer, and an exterior heating bath, there was charged under agitation 520 grams (also referred to as "g") of DMF (Mallinckrodt), 264 grams of MEK (Mallinckrodt), 200 grams of tertiary butyl alcohol (Arco), and 0.5 grams of MYRJ-53 (an ethoxylated stearic acid produced by ICI). Once a uniform solution was obtained, 15 grams of poly(acrylic acid) (B.F. Goodrich Carbopol® 940NF which is a homopolymer having a molecular weight of 1,250,000) powder were introduced by pouring directly into the reactor. The reactor was heated to 50°C and maintained at this temperature for one hour while under agitation at 2,000 RPM. Thereafter, the reactor was cooled to room temperature and the product discharged through a 10 micron polypropylene filter cartridge. A uniform colloidal dispersion was obtained. 180.02 grams of the filtered product was transferred to a Waring blender and was blended with 10.01 grams of 2,4,4'-trichloro-2-hydroxydiphenyl ether (Ciba Geigy Irgasan® DP300) for two minutes. The antimicrobial agent dissolved completely into the organic medium of colloidal dispersion to yield a homogeneous fluid.

### Example 2.

Eight French Percuflex® catheters (made of ethylene-vinyl acetate copolymer) were cut into 6-inch stents. The stents were wiped with Freon, air dried for 5 minutes. They were then dipped into a coating bath containing a polyisocyanate primer (Polyslip® Coating P-106 produced by Union Carbide) for 10 seconds and followed by drying in a forced air oven at 65°C for 1Q minutes. The stents were subsequently dipped in another coating bath containing the coating solution prepared in Example 1 for one second and followed by drying in a forced air oven at 65°C for 1 hour. The finished coating was uniform.

### Example 3.

This example illustrates the method for the measurement of stationary coefficient of friction (COF) of the catheter in the presence of water. A sliding block test was used for this measurement where a rectangular block wrapped in a wet membrane is placed on the surfaces of two parallel mounted catheters in the presence of distilled water. The platform on which the catheters rest is tilted slowly until the block begins to slide. The angle ø is measured. The stationary COF is calculated as the tan ø. The catheters are then subjected to mechanical abrasion by pushing and pulling the wet catheter through a tightly fitted silicone elastomer grommet (having a diameter 10% smaller than the outside diameter of the catheter) for 10 cycles or more. The abraded catheters are then measured again to give the COF after abrasion. The COF for the catheters coated in Example 2 before and after ten abrasions were found to be 0.04 and 0.04, respectively. The COF for the uncoated Percuflex stent was measured to be about 1.0.

### Example 4.

This example illustrates a biostatic coating formulation where a hydrophilic polymeric complex is used in conjunction with 2,4,4'-trichloro-2'-hydroxydiphenyl ether. A mixture consisting of 250 grams of the colloidal dispersion prepared in Example 1, 107.5 grams of Polyslip® Coating S-701 (a mixture of DMF-MEK-tBOH produced by Union Carbide), and 3.61 grams of 2,4,4'-trichloro-2-hydroxydiphenyl ether was mixed in a Waring blender at 30°C for two minutes to yield a uniform dispersion. Fourteen French Percuflex catheters were cut into 10-inch stents. The stents were cleaned with isopropyl alcohol and air dried for ten minutes. The clean stents were dipped into a coating bath containing Polyslip Coating P-106 for 30 seconds and followed by drying in a forced air oven at 65°C for 20 minutes. The stents were subsequently dipped into another coating bath containing the above-made solution for one second and followed by drying in a forced air oven at 65°C for 1 hour. The stents were dipped again in a third coating bath containing a 5% aqueous solution of Polyox® WSRN-80 (a poly(ethylene oxide) having a molecular weight of 200,000) for 1 second and followed by drying in a forced air oven at 65°C for 12 hours. The coating was uniform and lubricious in the presence of water. The COF in the presence of water measured before and after 100 abrasions were found to be 0.07 and 0.1, respectively. COF for the uncoated catheter was 1.0.

### Example 5.

Example 4 was repeated with the exception that (1) the quantity of 2,4,4'-trichloro-2-hydroxydiphenyl ether used was 18.8 grams instead of 3.61 grams, and (2) the third coating bath contained a 0.75% aqueous solution of PVP K-90 (poly(vinyl pyrrolidone) having a molecular weight of 700,000 produced by ISP) and the dipping time was 10 minutes instead of one second. The finished coating was uniform and lubricious in the presence of water. The COF in the presence of water measured before and after 100 abrasions were found to be 0.14 and 0.17, respectively. COF for the uncoated catheter was 1.0.

### Example 6.

Example 5 was repeated with the exception that (1) the quantity of 2,4,4'-trichloro-2-hydroxydiphenyl ether used was 13 grams, (2) the antimicrobial agent was first dissolved in the solvent mixture before mixing with the poly(acrylic acid) colloidal dispersion, and (3) the poly(vinyl pyrrolidone) concentration was increased from 0.75 to 1.0% by weight and the bath also contained 20 ppm of chlorine in the form of Clorox® as a preservative. The colloidal dispersion containing both poly(acrylic acid) and the antimicrobial agent showed a Brookfield viscosity of 3.7 centistokes and an average particle size of 1.2 microns. The finished coating was uniform and lubricious in the presence of water.

### Example 7.

The degree of lubricity and the abrasion resistance of the coating prepared in Example 6 was measured by a force gauge described below. The frictional force generated in pulling a coated catheter through a circular opening punched in a silicone membrane in the presence of water, where the inside diameter of the opening is slightly less than the outside diameter of the catheter to produce a tight grip during pulling, is a measure of the surface lubricity. The lower the frictional force the greater the lubricity, and vice versa. Measurement is made using a device capable of pulling the catheter through the silicone grommet at a constant speed of 4.5 inches per minute. Using this method, the frictional force values for the coated catheter before and after 100 abrasions, and the uncoated catheter were found to be 4.2, 10.7, and 39.5 grams, respectively. These results indicate a high degree of lubricity of the coated catheters.

### Example 8.

Example 6 was repeated with the exception that Polyslip Coating P-106 was replaced with a higher molecular weight polyisocyanate which was prepared from diphenylmethane diisocyanate and a high molecular weight polyester polyol having an isocyanate equivalent weight of 225 and an isocyanate content of 18.7%. The finished coating was uniform and lubricious in the presence of water. The frictional force values for the coated catheter before and after 100 abrasions, and the uncoated catheter were found to be 4.0, 3.3, and 39.5 grams, respectively.

### Example 9.

Example 4 was repeated with the exception that the Polyslip Coating P-106 was substituted with a polyisocyanate derived from toluene diisocyanate and polyol having a equivalent weight of 525 and isocyanate content of 8%. The finished coating was uniform and lubricious in the presence of water. Frictional force values for the coated catheter before and after 100 abrasions, and the uncoated catheter were found to be 12.2, 14.9, and 28.7, respectively.

### Example 10.

Two pieces of 11.5 inches, 14 French size Percuflex catheters were cleaned with isopropyl alcohol and air dried. The clean catheters were dipped into a coating bath, containing 1% of a vinyl chloride copolymer (UCAR® VMCA produced by Union Carbide), 3.5% of 2,4,4'-tichloro-2'-hydroxydiphenyl ether, and 95.5% of ethyl acetate (Mallinckrodt) all by weight percent, for 30 seconds. The wet catheters were dried in a forced air oven at 65°C for 30 minutes. They were then dipped into a second coating bath containing 0.5% of Polymer JR® (a quaternized cellulosic polymer produced by Union Carbide), 5% of isopropyl alcohol, and 95% of water all by weight percent. The catheters were baked in a forced air oven at 65°C for 1 hr. The coating was uniform. The coated catheter showed a contact angle with distilled water of 36°C in comparison to 58°C for the uncoated control. The COF for the coated and uncoated catheters were found to be 0.17 and 0.67, respectively.

### Example 11.

This example demonstrates the excellent bioefficacies of the biostatic coating composition of this invention against some common infectious bacteria using the zone of inhibition method. The larger the zone the greater the potency of the antimicrobial agent in controlling a given bacterium. The results are shown in Table I.

**Table I**

| Coating Type | Antimicrobial | Zone of Inhibition, mm | | |
|---|---|---|---|---|
| | Agent, % by wt | S. aureus | E. coli | P. mirabilis |
| Example 6 | 1 | 35.3 | 7 | |
| Example 6 | 2.5 | >40 | 32 | 13.1 |
| Example 6 | 3.5 | >40 | 41.8 | 18.2 |
| Example 8 | 3.5 | >40 | 27 | 16.3 |
| Uncoated | 0 | 0 | 0 | 0 |

### Example 12.

This example illustrates the nonhemolytic nature of the biostatic coatings containing 2,4,4'-trichloro-2'-hydroxydiphenyl ether. In vitro hemolysis test on a hydrogel biostatic coating containing different levels of the antimicrobial agent was tested by NAmSA® of Northwood, OH, using rabbit blood containing EDTA. Duplicate samples were tested in each case, and the result is reported as the mean value of the two tests. The results are shown in Table II.

**Table II**

| Coating Type | Antimicrobial % | Hemolysis % |
|---|---|---|
| Example 9 | 0 | 0%, non-hemolytic |
| Example 9 | 0.1 | 0%, non-hemolytic |
| Example 9 | 1 | 0%, non-hemolytic |
| Example 9 | 5 | 0%, non-hemolytic |

### Example 13.

Examples 13 to 15 illustrate the long-term bioefficacy of the biostatic coating of this invention in controlling the growth of some common infectious bacteria using different bioassays. The long-term bioefficacy of the biostatic coating prepared according to Example 6 was studied by following the zone of inhibition in agar culture challenged with 10⁵ CFU (Colony Forming Units) of a given bacterium. The same catheter specimen, which was sterilized with ethylene oxide, was transferred daily into a fresh culture challenged with the same bacterium until no bioefficacy was observed. The test was terminated at the end of 30 days regardless of the bioefficacy observed on the 30th day. The results are shown in Table III and table IV.

**Table III**

| ZONE OF INHIBITION DATA FOR E. COLI IN BIOASSAY EXAMPLE 13 | | | | |
|---|---|---|---|---|
| Sample | Zone of Inhibition, mm | | Discoloration | |
| | Day 1 | Day 30 | Biofilm Observed | Observed |
| Uncoated catheter | 0 | 0 | yes | yes |
| Coated but no antimicrobial agent | 0 | 0 | yes | yes |
| Coated with antimicrobial agent | 16 | 15 | no | no |

**Table IV**

| ZONE OF INHIBITION DATA FOR STAPH AUREUS IN BIOASSAY EXAMPLE 13 | | | | |
|---|---|---|---|---|
| Sample Day 1 | Zone of Inhibition, mm | | Discoloration | |
| | Day 30 | Biofilm | Observed | Observed |
| Uncoated catheter | 0 | 0 | yes | yes |
| Coated but no antimicrobial agent | 0 | 0 | yes | yes |
| Coated with antimicrobial agent | 16 | 16.3 | no | no |

### Example 14.

Example 13 was repeated with the exception that all sterilized catheter samples were incubated in trypticase soy broth for 24 hrs at 37°C before they were challenged with the infectious bacteria. The test was terminated at the end of 30 days regardless of the bioefficacy observed on the 30th day. The results are shown in Table V and Table VI.

**Table V**

| ZONE OF INHIBITION DATA FOR E. COLI IN BIOASSAY EXAMPLE 13 | | | | |
|---|---|---|---|---|
| Sample | Zone of Inhibition, mm | | Discoloration | |
| | Day 1 | Day 30 | Biofilm Observed | Observed |
| Uncoated | 0 | 0 | Yes | Yes |
| Coated but no antimicrobial agent | 0 | 0 | Yes | Yes |
| Coated with antimicrobial agent | 16.3 | 15.7 | No | No |

**Table VI**

| ZONE OF INHIBITION DATA FOR STAPH AUREUS IN BIOASSAY EXAMPLE 14 | | | | |
|---|---|---|---|---|
| Sample | Zone of Inhibition, mm | | Discoloration | |
| | Day 1 | Day 30 | Biofilm Observed | Observed |
| Uncoated | 0 | 0 | Yes | Yes |
| Coated but no antimicrobial agent | 0 | 0 | Yes | Yes |
| Coated with antimicrobial agent | 16 | 16 | No | No |

### Example 15.

The sterile catheters sections used in Example 13 were placed in three separate sealed flasks containing saline. The samples were incubated at 37°C in a shaker incubator. At three-day intervals, samples were removed for measuring zone of inhibition following the protocol described in Example 13. The rest of the catheter sections were transferred to flasks containing fresh saline, returned to the shaker incubator, and the experiment continued. The experimental results measured up to 30 days are compiled in Table VII and VIII.

**Table VII**

| ZONE OF INHIBITION DATA FOR E. COLI IN BIOASSAY EXAMPLE 15 | | | | |
|---|---|---|---|---|
| Sample | Zone of Inhibition, mm | | Discoloration | |
| | Day 1 | Day 30 | Biofilm Observed | Observed |
| Uncoated | 0 | 0 | Yes | Yes |
| Coated but no antimicrobial agent | 0 | 0 | Yes | Yes |
| Coated with antimicrobial agent | 14 | 16 | No | No |

**Table VIII**

| ZONE OF INHIBITION DATA FOR STAPH AUREUS IN BIOASSAY EXAMPLE 15 | | | | |
|---|---|---|---|---|
| Sample | Zone of Inhibition, mm | | Discoloration | |
| | Day 1 | Day 30 | Biofilm Observed | Observed |
| Uncoated | 0 | 0 | Yes | Yes |
| Coated but no antimicrobial agent | 0 | 0 | Yes | Yes |
| Coated with antimicrobial agent | 17 | 16 | No | No |

### Example 16

Examples 16-19 illustrate the preparation and application of an one-step antimicrobial coating composition containing 2,4,4'-trichloro-2-hydroxydiphenyl ether on a variety of medical devices.

Into an one-liter size Warring blender was charged 566.2 g of diacetone alcohol. While under mixing, there was added 1.58 g of poly(vinyl pyrrolidone) (Sigma poly(vinyl pyrrolidone)-360, Lot. 123-42-3), and 1.16 g of UCAR® VMCA, and the mixture was blended for 5 minutes to yield a uniform solution. The solution was then transterred to a glass jar containing 21 g of the antimicrobial agent which was subsequently roll milled to give a uniform solution.

### Example 17

Four pieces of latex Foley catheters ( 17 French produced by Baxter Healthcare Corporation) were wiped with IPA and air dried. The cleaned catheters were dipped into a coating solution prepared according to Example 16 for 30 sec., air-dried for 1 min., and then dried further in a foreed air oven at 85° C for 3 hrs. A clean, uniform coating was obtained. The finished catheters were sterilized with the standard ethylene oxide process and showed no change in physical characteristics.

### Example 18

Example 17 was repeated with the exception that the latex Foley catheters were replaced with a set of PVC endotracheal tubes and the drying was done at 90 instead of 85°C. The sterilized, coated endotracheal tubes were clear and uniform.

### Example 19

Example 17 was repeated again with the exception that the latex Foley catheters were replaced with a set of urinary stents ( 14 French Percuflex stents produced by Boston Scientific Corporation) and the drying was conducted at 65°C for 3 hrs. The sterilized, coated Percuflex stents were uniform and smooth.

### Example 20

Unexpectedly, It has been found that the biostatic hydrophilic coating of this invention also reduces encrustation on medical device exposed to human urine which was challenged with Proteus Mirabilis in an in-vitro model. This is demonstrated by the following example.

Into each of six sulture tubes there was added 12 ml of rehydrated urine (Rrichem®), 100 ul of 100 Klett Proteus Mirabilis, and a 3 inch long, 17 French latex Foley catheter piece. Three of the sterile catheter pieces had been coated with the biostatic coating according to Example 6, and the other three uncoated. All six culture tubes were placed in a 37°C incubator and incubated for 24 hrs. Thereafter, the culture tubes were removed from the incubator and examined visually for degrees of encrustation. All three uncoated catheter pieces showed different degrees of encrustation. On the other hand, all coated catheter pieces showed no visible encrustation on the surfaces.

Although the invention has been described with respect to specific aspects, those skilled in the art will recognize that other aspects of the invention are intended to be within the scope of the claims which follow. For example, the antimicrobial agent may also be incorporated in hydrophobic coatings or other coatings which are not hydrophilic or lubricious.

## Claims

1. A medical device having a hydrophilic coating thereon, said hydrophilic coating comprising:
(i) a layer of a hydrophilic polymer adhered to a surface of said medical device; and
(ii) an antimicrobial agent dispersed within said layer, said antimicrobial agent selected from the group consisting of; a halogenated 2-hydroxy diphenyl ether, a halogenated 2-acyloxy diphenyl ether or mixtures thereof.

2. The medical device of claim 1 wherein said coating further comprises a binder polymer.

3. The medical device of claim 1 wherein the antimicrobial agent is 2,4,4'-trichloro-2'-hydroxydiphenyl ether or a homologue or derivative thereof.

4. The medical device of claim 1 wherein the hydrophilic polymer is a naturally-occurring water-soluble polymer or derivative thereof.

5. The medical device of claim 1 wherein the hydrophilic polymer is a synthetic water-soluble polymer or a derivative thereof.

6. The medical device of claim 1 wherein the hydrophilic polymer is selected from the group consisting of poly(acrylic acid), cellulosic polymers or mixtures thereof.

7. The medical device of claim 1 wherein the hydrophilic polymer is comprised in a blend of at least two polymers comprising at least one water-soluble polymer.

8. In a process for applying a hydrophilic coating to a surface of a substrate comprising contacting the surface with:
(i) a hydrophilic polymer composition comprising a hydrophilic polymer; and
(ii) a binder polymer composition comprising a binder polymer which is capable of bonding to the surface of the substrate and the hydrophilic polymer; the improvement wherein at least one of the binder polymer composition or the hydrophilic ploymer composition further comprises an antimicrobial agent selected from the group consisting of a halogenated 2-hydroxy diphenyl ether, a halogenated 2-acyloxy diphenyl ether or mixtures thereof.

9. The process of claim 7 wherein an amount of antimicrobial agent is employed which is effective for controlling the growth of microorganisms.

10. The process of claim 8 wherein at least one of the hydrophilic polymers or the binder polymer is formed by applying monomers or prepolymers of said hydrophilic polymer or binder polymer to said substrate and polymerizing the monomer or prepolymer to form said hydrophilic polymer or binder polymer.
